Europäisches Patentamt

(19) European Patent Office

Office européen des brevets

(11) Numéro de publication : **0 007 895**
**B1**

(12) **FASCICULE DE BREVET EUROPÉEN**

(45) Date de publication du fascicule du brevet :
22.06.83

(51) Int. Cl.³ : **A 61 K   9/52**, A 61 K   9/00

(21) Numéro de dépôt : **79870017.5**

(22) Date de dépôt : **16.07.79**

(54) Nanoparticules biodégradables, compositions pharmaceutiques les contenant et procédé pour leur préparation.

(30) Priorité : **19.07.78 BE 189366**

(43) Date de publication de la demande :
**06.02.80 Bulletin 80/03**

(45) Mention de la délivrance du brevet :
**22.06.83 Bulletin 83/25**

(84) Etats contractants désignés :
**BE CH DE FR GB IT LU NL SE**

(73) Titulaire : **Couvreur, Patrick**
**9, Avenue Duc Wenceslas**
**B-1300 Wavre (BE)**

**Roland, Michel**
**38 Rue Tomberg**
**B-1150 Bruxelles (BE)**

**Speiser, Peter**
**26 Wassbargerstrasse**
**CH-8127 Forch (CH)**

(72) Inventeur : **Couvreur, Patrick**
**9, Avenue Duc Wenceslas**
**B-1300 Wavre (BE)**
Inventeur : **Roland, Michel**
**38 Rue Tomberg**
**B-1150 Bruxelles (BE)**
Inventeur : **Speiser, Peter**
**26 Wassbargerstrasse**
**CH-8127 Forch (CH)**

(74) Mandataire : **Plucker, Guy**
**OFFICE KIRKPATRICK 4 Square de Meeûs**
**B-1040 Bruxelles (BE)**

(56) Documents cités :
FR A 1 572 106
GB A 1 516 348
JOURNAL OF PHARMACEUTICAL SCIENCES no.
12, décembre 1976, vol. 65 G. BIRRENBACH et
P. P. SPEISER : « Polymerized Micelles and their
use as adjuvants in Immunology » pages 1763-
1766

(56) Documents cités :
CHEMICAL  ABSTRACTS, vol. 83, no. 3 11-08-
1975, page 305, abrégé 48196j Columbus, Ohio,
USA.
CHEMICAL ABSTRACTS, vol. 88, no. 18, mai
1978, page 322, abrégé 126303m Columbus, Ohio,
USA P. COUVREUR et al. : « Nanocapsules : a
new type of lysosomotropic carrier ».

CHEMICAL ABSTRACTS, vol. 88, no. 24, 12-06-1978, page 426, abrégé 177057s Columbus, Ohio, USA J.J. MARTY et al. : « Nanoparticles a new colloidal drug delivery system ».
CHEMICAL ABSTRACTS, vol. 89, no. 8, 21-08-1978, page 334, abrégé 65167s Columbus, Ohio, USA J. KREUTER : « Nanoparticles and nanocapsules. New dosage forms in the nanometer size range ».

Le dossier contient des informations techniques présentées postérieurement au dépôt de la demande et ne figurant pas dans le présent fascicule.

# Nanoparticules biodégradables, compositions pharmaceutiques les contenant et procédé pour leur préparation

La présente invention concerne des particules submicroscopiques formées d'un cyanoacrylate d'alkyle polymérisé et contenant une substance biologiquement active, leur préparation et leur application.

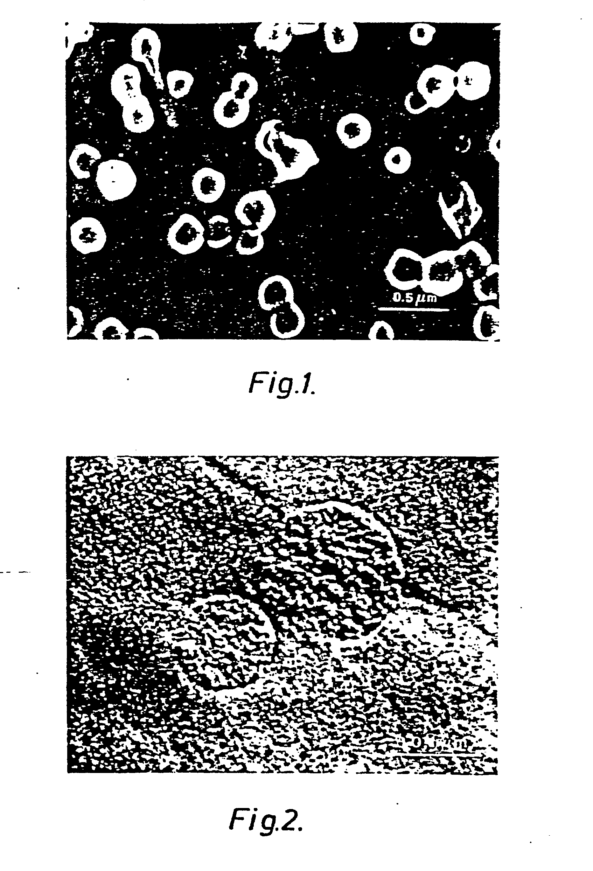

Au microscope électronique (figures 1 et 2), les particules submicroscopiques suivant l'invention se présentent sous la forme d'une pelote formée d'un réseau polymérique très dense, sensiblement sphérique d'un diamètre inférieur à 500 nanomètres et, de préférence, inférieur à 200 nanomètres ; elles sont formées par la polymérisation d'un cyanoacrylate d'alkyle où le terme « alkyle » désigne un radical alkyle inférieur contenant de 1 à 4 atomes de carbone et plus particulièrement le radical méthyle. Les particules de l'invention contiennent au sein de leur réseau filamenteux une substance biologiquement active qui peut être par exemple une substance médicamenteuse à usage humain ou vétérinaire ou un produit pour le diagnostic. Comme substance médicamenteuse, on peut citer plus particulièrement les produits chimiques doués de propriétés pharmacologiques et, par exemple, les substances antimitotiques ou antinéoplasiques comme le méthotrexate, l'actinomycine D, l'adriamycine, la daunorubicine, la bléomycine et la vincristine ou les substances antibiotiques comme les pénicillines, les céphalosporines et l'acide nalidixique, les antibiotiques du type aminoglycoside et ceux de la famille de la virginiamycine et les substances hormonales, notamment les hormones stéroïdiennes. Ces substances médicamenteuses peuvent être notamment des composés chimiques à haut poids moléculaire comme l'insuline et l'héparine et l'expression « substance médicamenteuse » comprend également des produits biologiques comme les antigènes, les allergènes, les enzymes, les protéines, les virus ou des constituants de virus, de bactéries ou de cellules. Les particules submicroscopiques suivant l'invention peuvent également contenir un produit pour le diagnostic comme par exemple la fluorescéine et la séralbumine humaine radio-active.

En médecine humaine ou vétérinaire, les particules submicroscopiques de l'invention peuvent être administrées avec un excipient adéquat par voie orale, sous-cutanée, intradermique, intramusculaire ou intraveineuse et leur diffusion dans les tissus les rend particulièrement intéressantes pour les traitements par voie générale.

On connaît déjà des particules de diamètre inférieur à 500 nanomètres formées d'une matière polymérisée, ainsi que leur préparation et leur application, notamment comme support d'une substance biologiquement active.

Ainsi, les brevets belges n° 808.034 et 839.748 décrivent des particules submicroscopiques formées de matières polymérisables comme les dérivés de l'acide acrylique ou méthacrylique, par exemple le méthacrylate de méthyle, le méthacrylate de butyle, le méthacrylamide ou un mélange de ces composés. Les particules submicroscopiques formées par polymérisation micellaire de ces différents monomères ont à la fois la propriété d'enrober totalement ou partiellement la substance biologiquement active et la propriété de former des solutions aqueuses colloïdales qui permettent une administration parentérale de ces particules ainsi chargées de substances biologiquement actives.

Cependant, les polymères des dérivés de l'acide acrylique ou méthacrylique décrits ou cités jusqu'ici pour la préparation de particules submicroscopiques contenant une substance biologiquement active sont substantiellement stables de sorte qu'ils subsistent longtemps tels quels dans les tissus ou dans la cavité où ils ont été administrés et ceci constitue un inconvénient, plus particulièrement dans le cas d'administration parentérale en médecine humaine.

La présente invention remédie à ce désavantage en utilisant comme matière de départ les cyanoacrylates d'alkyle dont les polymères, d'ailleurs déjà utilisés en chirurgie comme adhésifs tissulaires et hémostatiques, sont nettement biodégradables.

Dans les dessins annexés :

Figure 1 est une photomicrographie (microscope électronique à balayage) montrant l'aspect morphologique des nanoparticules de polyméthylcyanoacrylates ;

Figure 2 est une photomicrographie (microscope électronique) montrant la structure interne des nanoparticules de polyméthylcyanacrylate après cryofracture ;

Figure 3 est un diagramme des cinétiques de libération de l'actinomycine D à partir de nanosphérules de polyalkylcyanoacrylate en milieu sérique (en pour-cent en ordonnées, le temps étant donné en heures en abscisses) et dans lequel :

— la courbe A concerne le polyméthylcyanoacrylate,

— la courbe B concerne un mélange 2 : 1 de polyméthyl- et polyéthylcyanoacrylate,

— la courbe C concerne un mélange 1 : 1 de polyméthyl- et polyéthylcyanoacrylate,

— la courbe D concerne un mélange 1 : 2 de polyméthyl- et polyéthylcyanoacrylate,

— la courbe E concerne le polyéthylcyanoacrylate.

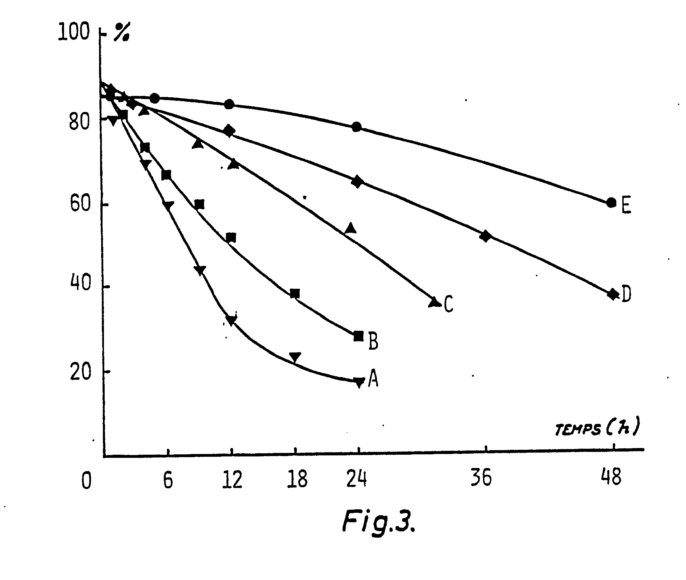

En conséquence, l'administration de particules submicroscopiques suivant l'invention est suivie d'une libération progressive de la substance biologiquement active en fonction de la biodégradabilité de la matière polymérisée. Comme on sait que la biodégrabilité des polycyanoacrylates d'alkyle dépend de la nature de la chaîne alkyle, il est possible de choisir le produit dont la forme polymérisée présente une biodégradabilité correspondant au programme fixé pour la libération de la matière biologiquement active. Ainsi, la cinétique de dégradabilité de ces nanoparticules en milieu sérique de même que la libération du médicament adsorbé peuvent être parfaitement contrôlées et programmées en fonction de l'effet thérapeutique recherché. Cet objectif peut être atteint par l'emploi de mélanges adéquats de nanosphères (figure 3).

Un autre avantage des cyanoacrylates d'alkyle sur les autres dérivés acryliques précédemment utilisés pour la préparation de particules submicroscopiques contenant une substance biologiquement active réside dans le procédé utilisable pour leur polymérisation. En effet, contrairement aux autres dérivés acryliques dont la polymérisation requiert un apport énergétique susceptible de nuire à la stabilité du principe actif adsorbé, les cyanoacrylates d'alkyle polymérisent particulièrement facilement sans un tel apport. Dans le procédé de préparation des particules submicroscopiques suivant l'invention, le monomère est ajouté à une solution aqueuse d'un agent tensio-actif, de préférence un agent tensio-actif non ionique comme par exemple le monolaurate de sorbitan polyoxyéthylé, soumise à une agitation énergique de manière à former une solution micellaire dont le pH est ajusté à une valeur inférieure à 7, et de préférence compris entre 2 et 3, avec un acide pharmacologiquement acceptable comme par exemple l'acide chlorhydrique, bromhydrique, iodhydrique, sulfurique, phosphorique, acétique, succinique ou lactique pour autant que ledit acide soit compatible avec les composants du milieu, c'est-à-dire qu'il n'agisse pas en sens contraire de l'action de l'un ou l'autre ingrédient du milieu et notamment de l'agent tensio-actif ; le cyanoacrylate d'alkyle polymérise à la température ordinaire ou même à température inférieure à la température ordinaire, la substance biologiquement active étant introduite dans le milieu soit avant l'introduction du monomère soit après polymérisation. Le pH du milieu règle à la fois la vitesse de polymérisation et le degré d'adsorption de la matière biologiquement active dans le cas où celle-ci peut se présenter sous forme ionisée.

On constate en effet que la polymérisation est ralentie par un abaissement du pH et que l'adsorption de la matière biologiquement active atteint un maximum lorsque celle-ci se présente sous sa forme la plus lipophile possible, c'est-à-dire non ionisée ou encore lorsque le pH du milieu correspond à la valeur $pK_a$ de la substance biologiquement active.

En pratique, on peut concilier ces deux exigences en effectuant d'abord la polymérisation à un pH permettant de maîtriser la réaction, c'est-à-dire de préférence à une valeur du pH comprise entre 2 et 3 et en effectuant l'adsorption après polymérisation en ajustant alors si nécessaire le pH du milieu à la valeur correspondant au $pK_a$ de la substance biologiquement active.

L'invention est illustrée par les exemples suivants qui n'en limitent pas la portée.

Exemple 1

Dans 45,5 ml d'eau distillée, on dissout 180 mg de monolaurate de sorbitan polyoxyéthylé, 4,5 ml d'acide chlorhydrique 0,1 N et un mg d'actinomycine D tritiée et on ajoute lentement sous agitation énergique 0,83 ml de cyanoacrylate de méthyle. L'agitation est maintenue pendant 30 minutes après complète addition du monomère.

La polymérisation qui s'effectue spontanément rend d'abord la suspension opalescente et lui confère ensuite un aspect laiteux. Le milieu de réaction est tamponné à pH 7 à l'aide de quelques gouttes de soude caustique normale et de 5 ml de tampon phosphate (50 ml de phosphate monopotassique 0,2 M et 30 ml d'hydroxyde de sodium 0,2 N portés à 200 ml) et centrifugé à 50.000 g.

Par le dosage de l'activité radio-active (scintillation liquide) dans le surnageant et dans le culot de centrifugation, on constate que la quantité d'actinomycine D fixée sur les particules correspond à 90 % de la quantité engagée avant polymérisation. L'examen des particules au microscope électronique après cryofracture révèle qu'elles ont une forme sensiblement sphérique de diamètre inférieur à 200 nanomètres. (Fig. 2)

Exemple 2

Dans 45,5 ml d'eau distillée, on dissout 180 mg de monolaurate de sorbitan polyoxyéthylé, 4,5 ml d'acide chlorhydrique 0,1 N et un mg d'actinomycine D tritiée et on ajoute lentement sous agitation énergique 0,83 ml de cyanoacrylate d'éthyle. L'agitation est maintenue pendant une heure après complète addition du monomère. Le milieu de réaction est filtré sur verre fritté dont les pores ont un diamètre compris entre 9 et 15 microns et le filtrat est tamponné à pH = 7 à l'aide de quelques gouttes de soude caustique normale et de tampon phosphate (50 ml de phosphate monopotassique 0,2 M et 30 ml d'hydroxyde de sodium 0,2 M portés à 200 ml) et centrifugé à 50.000 g.

Par dosage de la radio-activité (scintillation liquide) dans le surnageant et dans le culot de centrifugation, on constate que la quantité d'actinomycine D fixée sur les particules correspond à 85 % de la quantité engagée. L'examen des particules au microscope électronique à balayage révèle une structure et une dimension identiques à celles des particules obtenues dans l'exemple 1.

Exemple 3

Dans 30 ml d'acide chlorhydrique 0,1 N, on dissout 300 mg de monolaurate de sorbitan polyoxyéthylé et un mg d'actinomycine D et on procède ensuite suivant la technique de l'exemple 1 avec 0,5 ml de cyanoacrylate de méthyle. Dans ces conditions, on obtient des particules comparables à celles obtenues dans l'exemple 1 mais présentant dans l'ensemble un diamètre compris entre 300 et 500 nanomètres.

Exemple 4

Dans 30 ml d'eau distillée, on dissout 3 ml d'acide chlorhydrique 0,1 N et, respectivement, 50, 100, 200, 300 et 700 mg de monolaurate de sorbitan polyoxyéthylé. On ajoute lentement et sous agitation énergique 0,5 ml de cyanoacrylate de méthyle et on procède comme dans l'exemple 1. En fin d'opération on obtient chaque fois des particules ne présentant aucune différence morphologique ou granulométrique par rapport aux particules obtenues à la fin de l'exemple 1.

Exemple 5

On procède suivant la technique décrite dans l'exemple 1 mais sans introduire l'actinomycine D. Dans ces conditions, on obtient des particules sans substance active et leur examen au microscope électronique après cryofracture révèle qu'elles ont une forme sensiblement sphérique de diamètre inférieur à 200 nanomètres et montre que leur structure interne est constituée d'un réseau polymérique très dense développant une surface spécifique importante. Ces nanosphères ne présentent pas d'enveloppe externe et ne sont pas conséquent pas assimilables à des nanocapsules (figure 2).

Exemple 6

Dans 45,5 ml d'eau distillée, on dissout 80 mg de monolaurate de sorbitan polyoxyéthylé, 4,5 ml d'acide chlorhydrique 0,1 N et on ajoute lentement sous agitation énergique 0,83 ml de cyanoacrylate de méthyle. L'agitation est maintenue pendant 30 minutes après complète addition de monomère. Le milieu est tamponné à pH 7 à l'aide de quelques gouttes de soude caustique normale et de 5 ml de tampon phosphate (50 ml de phosphate monopotassique 0,2 M et 30 ml d'hydroxyde de sodium 0,2 N portés à 200 ml) et on y ajoute sous agitation un mg d'actinomycine D tritiée. Le milieu est maintenu sous agitation pendant une demi-heure et est ensuite centrifugé à 50.000 g.

Par dosage (scintillation liquide) de l'actinomycine D dans le surnageant et dans le culot de centrifugation, on constate que la quantité d'actinomycine D fixée sur les particules correspond à 66 % de la quantité engagée.

Exemple 7

Dans 50 ml d'eau distillée, on dissout 250 mg de monolaurate de sorbitan polyoxyéthylé, 5 ml d'acide chlorhydrique 0,1 N et 10 mg de fluorescéine et on ajoute lentement sous agitation énergique 0,6 ml de cyanoacrylate d'éthyle. L'agitation est maintenue pendant 30 minutes après complète addition du monomère. La suspension est alors diluée à 200 ml par addition d'eau distillée. Le milieu est ensuite centrifugé à 50.000 g.

Par dosage fluorométrique de la fluorescéine dans le surnageant et dans le culot de centrifugation, on observe que la quantité de fluorescéine fixée sur les particules est de l'ordre de 65-70 % de la quantité engagée.

Exemple 8

Dans 45 ml d'eau distillée, on dissout 200 mg de monolaurate de sorbitan polyoxyéthylé, 5 ml d'acide chlorhydrique 0,1 N et 5 mg de méthotrexate et on ajoute lentement sous agitation énergique 0,83 ml de cyanoacrylate de méthyle. L'agitation est maintenue pendant 30 minutes après complète addition de monomère. 20 ml de la suspension sont tamponnés à pH 7 à l'aide de quelques gouttes de soude caustique normale et de tampon phosphate (50 ml de phosphate monopotassique 0,2 M et 30 ml d'hydroxyde de sodium 0,2 N portés à 200 ml) de manière à porter la suspension à 25 ml. Le milieu est ensuite centrifugé à 50.000 g.

Par dosage fluorométrique du méthotrexate dans le surnageant et dans le culot de centrifugation, on constate que la quantité de méthotrexate fixée sur les particules correspond à environ 25 % (± 5 %) de la quantité engagée.

Exemple 9

Dans 50 ml d'eau distillée stérile et apyrogène on dissout 200 mg de monolaurate de sorbitan polyoxyéthylé, 5 ml d'acide chlorhydrique 0,1 N et 5 mg d'actinomycine D et on ajoute lentement sous agitation énergique 0,83 ml de cyanoacrylate de méthyle. Le milieu est porté à pH 7 à l'aide de quelques gouttes de soude caustique normale et de tampon phosphate (50 ml de phosphate monopotassique 0,2 M et 30 ml d'hydroxyde de sodium 0,2 N portés à 200 ml). Le milieu est alors stérilisé et filtré et distribué dans des ampoules en verre à raison de 0,5 mg d'actinomycine D par ampoule. Les ampoules sont bouchées hermétiquement et stockées à l'abri de la lumière. Elles contiennent des doses unitaires d'actinomycine D convenant à l'administration parentérale locale ou à la perfusion intraveineuse après reconstitution dans un diluant isotonique convenant à l'une quelconque de ces administrations.

Exemple 10

Dans 45,5 ml d'eau distillée, on dissout 180 mg de monolaurate de sorbitan polyoxyéthylé et 4, 5 ml d'acide chlorhydrique 0,1 N. A cette solution, on ajoute 500 mg d'amylose. Cette suspension est ensuite chauffée à environ 80 ° C jusqu'à dissolution complète de l'amylose. La solution est ensuite refroidie et filtrée, 50 ml de propylène glycol sont ensuite mélangés à la solution précédente et 0,500 ml de cyanoacrylate de butyle sont ajoutés sous agitation. L'agitation est maintenue pendant quatre heures après complète addition du monomère. Le milieu est ensuite tamponné à

pH = 7 à l'aide de quelques gouttes de soude caustique normale et 10 ml de tampon phosphate (50 ml de phosphate monopotassique 0, 2 M et 30 ml d'hydroxyde de sodium 0,2 N portés à 200 ml).

Exemple 11

Dans 45,5 ml d'eau distillée, on dissout 180 mg de monolaurate de sorbitan polyoxyéthylé et 4,5 ml d'acide chlorhydrique 0,1 N. On ajoute lentement sous agitation énergique 0,83 ml de cyanoacrylate d'alkyle (méthyle ou éthyle). L'agitation est maintenue pendant 1 heure après complète addition du monomère. 250 UI d'insuline sont ensuite dissoutes dans la suspension de nanoparticules ainsi formées. Après 1 heure de contact, le milieu de réaction est tamponné à pH = 7 à l'aide de quelques gouttes de soude caustique normale et de 5 ml de tampon phosphate (50 ml de phosphate monopotassique 0,2 M et 30 ml d'hydroxyde de sodium 0,2 N portés à 200 ml) et centrifugé à 50.000 g.

Par dosage radioimmunologique de l'insuline dans le surnageant et dans le culot de centrifugation, on constate que la quantité d'insuline fixée sur les nanoparticules correspond à 80 % de la quantité mise en œuvre dans le cas des nanoparticules de polyméthylcyanoacrylate et à 75 % de cette même quantité dans le cas des nanoparticules de polyéthylcyanoacrylate.

Après administration sous-cutanée de ces dernières à des rats WHISTAR R mâles rendus préalablement diabétiques par injection intrapéritonéale d'alloxane, un effet hypoglycémiant a pu être obtenu durant 24 heures.

Exemple 12

Dans 45,5 ml d'eau distillée, on dissout 180 mg de monolaurate de sorbitan polyoxyéthylé, 4,5 ml d'acide chlorhydrique 0,1 N et 17,5 mg de vinblastine marquée au tritium. On ajoute sous agitation énergique 0,83 ml de cyanoacrylate d'éthyle. L'agitation est maintenue pendant 1 heure après complète addition du monomère.

Le milieu de réaction est tamponné à pH = 7 à l'aide de quelques gouttes de soude caustique normale et de 5 ml de tampon phosphate (50 ml de phosphate monopotassique 0,2 M et 30 ml d'hydroxyde de sodium 0,2 N portés à 200 ml) et centrifugé à 50 000 g.

Par dosage de la radio-activité (scintillation liquide) dans le surnageant et dans le culot de centrifugation, on constate que la quantité de vinblastine fixée sur les particules correspond à environ 70 % de la quantité engagée avant polymérisation.

Lorsque la vinblastine est associée à des nanoparticules de polyéthylcyanoacrylate, la distribution corporelle de ce médicament se trouve sensiblement modifiée après administration intraveineuse à des rats WHISTAR R mâles. Une concentration tissulaire très supérieure à celle du produit pur est observée au niveau du foie, de la rate, des poumons et du muscle.

Exemple 13

Dans 45,5 ml d'eau distillée, on dissout 180 mg de monolaurate de sorbitan polyoxyéthylé, 4,5 ml d'acide chlorhydrique 0,1 N et 40 mg de chlorhydrate de Levamisole. On ajoute lentement et sous agitation énergique 0,750 ml de cyanoacrylate de méthyle. L'agitation est maintenue pendant une demi-heure après complète addition du monomère. Le milieu de réaction est ensuite tamponné à pH = 7 à l'aide de quelques gouttes de soude caustique normale et de 5 ml de tampon phosphate. Après 24 heures, la suspension est centrifugé à 50 000 g.

Par dosage spectrophotométrique (à 212 nm), du Levamisole dans le surnageant et dans le culot de centrifugation, on constate que la quantité de principe actif fixée sur les nanoparticules correspond à 25 % de la quantité engagée.

Exemple 14

Dans 45,5 ml d'eau distillée, on dissout 180 mg de monolaurate de sorbitan polyoxyéthylé, 4,5 ml d'acide chlorhydrique 0,1 N et 50 mg de Pénicilline V potassique. On ajoute lentement et sous agitation énergique 0,800 ml de cyanoacrylate de méthyle. L'agitation est maintenue pendant une demi-heure après complète addition du monomère. Le milieu de réaction est ensuite tamponné à pH = 7 à l'aide de quelques gouttes de soude caustique normale et de 5 ml de tampon phosphate. La suspension est ensuite centrifugée à 50 000 g.

Par dosage spectrophotométrique (à 325 nm après dégradation de la pénicilline à l'imidazole et réaction avec $HgCl_2$) de la Pénicilline V dans le surnageant, on constate que la quantité de principe actif fixée sur les nanoparticules correspond à environ 50 % de la quantité engagée.

**Revendications**

1. Particules biodégradables d'un diamètre inférieur à 500 nanomètres formées par la polymérisation micellaire d'un cyanoacrylate d'alkyle où le terme « alkyle » désigne un radical alkyle inférieur de 1 à 4 atomes de carbone et contenant une substance biologiquement active.

2. Particules biodégradables suivant la revendication 1, caractérisées en ce que leur diamètre est inférieur à 200 nanomètres.

3. Particules biodégradables suivant l'une quelconque des revendications 1 et 2, caractérisées en ce qu'elles sont formées par la polymérisation de cyanoacrylate de méthyle.

4. Particules biodégradables suivant l'une quelconque des revendications 1 à 3, caractérisées en ce que la substance biologiquement active est un produit doué de propriétés antimitotique, antinéoplasique, antibiotique ou hormonale, un virus, un constituant de virus, de bactérie ou de cellule, un antigène, un allergène ou un enzyme.

5. Composition pharmaceutique contenant des particules biodégradables suivant l'une quelconque des revendications 1 à 4, avec un excipient pour l'administration orale, sous-cutanée, intradermique, intramusculaire ou intraveineuse.

6. Procédé de préparation d'une composition pharmaceutique contenant des particules biodégradables suivant l'une quelconque des revendications 1 à 4, caractérisé en ce qu'il consiste :

— à préparer une solution ou solution colloïdale aqueuse d'une substance biologiquement active, contenant en outre un agent tensio-actif et ajustée à un pH inférieur à 7 avec un acide pharmaceutiquement acceptable,

— à ajouter sous agitation à cette solution ou solution colloïdale aqueuse un cyanoacrylate d'alkyle où le terme « alkyle » désigne un radical alkyle inférieur de 1 à 4 atomes de carbone,

— à poursuivre l'agitation jusqu'à ce qu'en substance tout le cyanoacrylate d'alkyle introduit dans le milieu réactionnel se soit transformé en particules submicroscopiques formées de polycyanoacrylate d'alkyle.

7. Procédé de préparation d'une composition pharmaceutique contenant des particules biodégradables suivant l'une quelconque des revendications 1 à 4, caractérisé en ce qu'il consiste :

— à préparer une solution aqueuse d'un agent tensio-actif ajustée à un pH inférieur à 7 avec un acide pharmaceutiquement acceptable,

— à ajouter sous agitation à cette solution aqueuse un cyanoacrylate d'alkyle où le terme « alkyle » désigne un radical alkyle inférieur de 1 à 4 atomes de carbone,

— à poursuivre l'agitation jusqu'à ce qu'en substance tout le cyanoacrylate d'alkyle introduit dans le milieu réactionnel se soit transformé en particules submicroscopiques formées de polycyanoacrylate d'alkyle,

— à ajouter à cette suspension de particules submicroscopiques une substance biologiquement active.

8. Procédé suivant l'une quelconque des revendications 6 et 7, caractérisé en ce que la dite solution ou solution colloïdale aqueuse est ajustée à un pH compris entre 2 et 3 avant qu'on y ajoute le cyanoacrylate d'alkyle.

9. Procédé suivant l'une quelconque des revendications 6 à 8, caractérisé en ce que, après la formation de la suspension de particules submicroscopiques contenant la dite substance biologiquement active, le pH de cette suspension est ajustée à 7.

**Claims**

1. Biodegradable particles with a diameter less than 500 nanometers formed by the micellar polymerisation of an alkyl cyanoacrylate in which the alkyl radical has 1 to 4 carbon atoms and containing a biologically active substance.

2. Biodegradable particles according to claim 1, characterised in that their diameter is below 200 nanometers.

3. Biodegradable particles according to any of claims 1 and 2, characterised in that they are formed by the polymerisation of methyl cyanoacrylate.

4. Biodegradable particles according to any of claims 1 to 3, characterised in that the biologically active substance is a product with antimitotic, antineoplastic, antibiotic or hormonal properties, a virus, a virus component, a bacterium component, a cell component, an antigen, an allergen or an enzyme.

5. A pharmaceutical composition containing biodegradable particles according to any of claims 1 to 4 and an excipient for orally, subcutaneously, intradermally, intramuscularly or intraveneously administering said composition.

6. A process for preparing a pharmaceutical composition comprising biodegradable particles according to any of claims 1 to 4, characterised by comprising the steps of

— preparing an aqueous solution or colloidal solution of a biologically active substance, also containing a surface active agent and adjusted to a pH value lower than 7 with a pharmaceutically acceptable acid,

— adding, with stirring, to said aqueous solution or colloidal solution an alkyl cyanoacrylate wherein the alkyl radical has 1 to 4 carbon atoms,

— stirring further until substantially all the alkyl cyanoacrylate introduced in the reaction medium has been transformed into submicroscopic particles comprising alkyl polycyanoacrylate.

7. A process for preparing a pharmaceutical composition comprising biodegradable particles according to any of claims 1 to 4, characterised by comprising the steps of

— preparing an aqueous solution of a surface active agent, wherein the solution is adjusted to a pH value lower than 7 with a pharmaceuticallyl acceptable acid,

— adding, with stirring, to said aqueous solution an alkyl cyanoacrylate, wherein the alkyl radical has 1 à 4 carbon atoms,

— stirring further until substantially all the alkyl cyanoacrylate introduced in the reaction medium has been transformed into submicroscopic particles comprised of alkyl polycyanoacrylate,

— adding to the resulting suspension of submicroscopic particles a biologically active substance.

8. A process according to any of claims 6 and 7, characterised in that said aqueous solution or colloidal solution is adjusted to a pH value in the range of 2 to 3 before the alkyl cyanoacrylate is added thereto.

9. A process according to any of claims 6 to 8, characterised in that, after the suspension of submicroscopic particles containing said biologically active substance has been formed, the pH of the suspension is adjusted to a value of 7.

**Ansprüche**

1. Bio-abbaubare Teilchen mit einem Durchmesser unter 500 Nanometer, gebildet durch

Mizellen-Polymerisation eines Cyanoacrylsäure-alkylesters, worin der Ausdruck « Alkyl » einen niederen Alkylrest mit 1 bis 4 Kohlenstoffatomen bedeutet, und enthaltend eine biologisch aktive Substanz.

2. Bio-abbaubare Teilchen gemäß Anspruch 1, dadurch gekennzeichnet, daß ihr Durchmesser unterhalb von 200 Nanometer ist.

3. Bio-abbaubare Teilchen gemäß einem der Ansprüche 1 und 2, dadurch gekennzeichnet, daß sie durch Polymerisation von Cyanoacrylsäure-methylester gebildet sind.

4. Bio-abbaubare Teilchen gemäß einem der Ansprüche 1 bis 3, dadurch gekennzeichnet, daß die biologisch aktive Substanz ein Produkt mit antimitotischen, antineoplasmischen, antibiotischen oder hormonalen Eigenschaften, ein Virus, ein Virus-, Bakterien- oder Zellenbestandteil, ein Antigen, ein Allergen oder ein Enzym ist.

5. Pharmazeutische Zusammensetzung, enthaltend bio-abbaubare Teilchen gemäß einem der Ansprüche 1 bis 4 mit einem Exzipienten zur oralen, subkutanen, intradermalen, intramuskulären oder intravenösen Verabreichung.

6. Verfahren zur Herstellung einer pharmazeuti-schen Zusammensetzung, enthaltend bio-abbau-bare Teilchen gemäß einem der Ansprüche 1 bis 4, dadurch gekennzeichnet, daß es darin besteht :
— eine wäßrige Lösung oder kolloidale wäßrige Lösung einer biologisch aktiven Substanz herzustellen, die außerdem ein oberflächenaktives Mittel enthält, und die mit einer pharmazeutisch annehmbaren Säure auf ein pH unterhalb von 7 eingestellt ist,
— unter Rühren zu dieser Lösung oder wäßrigen, kolloidalen Lösung einen Cyanoacrylsäure-alkylester, worin der Ausdruck « Alkyl » einen niedrigen Alkylrest mit 1 bis 4 Kohlenstoffatomen bedeutet, zuzugeben,
— das Rühren fortzusetzen, bis im wesentlichen das ganze in das Reaktionsmilieu eingeführte Alkyl-cyanoacrylat in submikroskopische Teilchen, welche aus Polyalkyl-cyanoacrylat gebildet sind, überführt ist.

7. Verfahren zur Herstellung einer pharmazeuti-schen Zusammensetzung, enthaltend bio-abbau-bare Teilchen gemäß einem der Ansprüche 1 bis 4, dadurch gekennzeichnet, daß es darin besteht :
— eine wäßrige Lösung eines oberflächenaktiven Mittels herzustellen, die auf ein pH unterhalb von 7 mit einer pharmazeutisch annehmbaren Säure eingestellt ist,
— unter Rühren zu dieser wäßrigen Lösung einen Cyanoacrylsäure-alkylester, worin der Ausdruck « Alkyl » einen niederen Alkylrest mit 1 bis 4 Kohlenstoffatomen bedeutet, zuzusetzen,
— das Rühren fortzusetzen, bis das ganze in das Reaktionsmilieu eingeführte Alkylcyanoacrylat in submikroskopische Teilchen, gebildet aus Polyalkyl-cyanoacrylat, umgewandelt ist,
— zu dieser Suspension submikroskopische Teilchen einer biologisch aktiven Substanz zuzusetzen.

8. Verfahren gemäß einem der Ansprüche 6 und 7, dadurch gekennzeichnet, daß die genannte Lösung oder wäßrige, kolloidale Lösung auf ein pH zwischen 2 und 3 eingestellt wird, bevor man das Alkyl-cyanoacrylat zusetzt.

9. Verfahren gemäß einem der Ansprüche 6 bis 8, dadurch gekennzeichnet, daß nach der Bildung der Suspension der submikroskopischen Teilchen, welche die genannte biologisch aktive Substanz enthalten, das pH dieser Suspension auf 7 eingestellt wird.

Fig.1.

Fig.2.

Fig.3.